# FASCICULE DE BREVET EUROPEEN

(11) **EP 4 164 486 B1**
(45) Date de publication et mention de la délivrance du brevet: **19.08.2026**
(21) Numéro de dépôt: 21742485.2
(22) Date de dépôt: 11.06.2021
(51) Int. Cl.: A61B 5/145, A61B 5/1486, A61B 5/1473, A61B 5/1495

(54) **PROCÉDÉ POUR DÉTERMINER UNE CONCENTRATION RÉELLE D'UN SUBSTRAT À L'AIDE D'UN ENSEMBLE DE BIOCAPTEURS AUTO-CALIBRES ET DISPOSITIF POUR LA MISE EN OEUVRE DU PROCÉDÉ**
VERFAHREN ZUR BESTIMMUNG EINER IST-KONZENTRATION EINES SUBSTRATS MIT EINEM ARRAY SELBSTKALIBRIERTER BIOSENSOREN UND VORRICHTUNG ZUR DURCHFÜHRUNG DES VERFARHENS
METHOD FOR DETERMINING AN ACTUAL CONCENTRATION OF A SUBSTRATE USING AN ARRAY OF SELF-CALIBRATED BIOSENSORS AND DEVICE FOR IMPLEMENTING THE METHOD

(30) Priorité: 12.06.2020 FR 2006175
(43) Date de publication de la demande: 19.04.2023
(73) Titulaire: UNIVERSITE GRENOBLE ALPES, 38400 Saint Martin d'Hères (FR); Centre National de la Recherche Scientifique, 75016 Paris (FR); Institut Polytechnique de Grenoble, 38031 Grenoble Cedex 01 (FR); Centre Hospitalier Universitaire Grenoble Alpes, 38700 La Tronche (FR)
(72) Inventeur: ZEBDA, Abdelkader, 38100 Grenoble (FR); CINQUIN, Philippe, 38330 Saint-Nazaire-les-Eymes (FR); MARTIN, Don, 38610 Gières (FR)
(74) Mandataire: Cabinet Chaillot
(86) Numéro de dépôt international: PCT/IB2021/055158
(87) Numéro de publication internationale: WO 2021/250627

(56) Documents cités:
- US-A- 5 168 046
- US-A1- 2007 299 617
- US-A1- 2010 094 110
- US-B2- 8 679 310

## Description

La présente invention porte sur un dispositif pour la mise en œuvre d'un procédé pour déterminer, de façon stable au cours du temps, une région dans laquelle se situe la concentration réelle dans un milieu, d'un substrat qui est susceptible d'oxydo-réduction avec une enzyme, à l'aide d'un ensemble de biocapteurs.

Le diabète est une maladie épidémique mondiale qui affectait 422 millions de personnes en 2014, contre 108 millions en 1980 et devrait être la septième cause de décès dans le monde si les tendances actuelles de morbidité se poursuivent [1,2,3]. En 2016, près de 12% de la population française était diagnostiqué avec un problème de diabète [4]. En plus d'augmenter le taux de mortalité, le diabète et ses complications infligent des pertes économiques considérables et des dépenses de sécurité sociale importantes (arrêt de travail, traitements, soins hospitaliers...) [4]. En effet, en France, le coût du traitement du diabète est d'environ 10 milliards d'euros, dont 80% liés au traitement des complications. Or ces complications pourraient justement être anticipées et/ou évitées avec des dispositifs fiables de mesure de la glycémie en temps réel [5].

L'émergence de capteurs de glucose a permis aux patients de gérer leurs niveaux d'insuline et donc de contribuer à limiter la mortalité du diabète. Les dispositifs de test de glucose traditionnels comprennent des capteurs de glucose basés sur des méthodes électrochimiques (glucomètre). Un biocapteur à glucose est constitué d'une électrode de travail à base de matériaux conducteurs associés à une enzyme capable de catalyser l'oxydation du glucose telle que la glucose oxydase (GOx) et la glucose déshydrogénase (GDH) et d'une contre-électrode qui peut être en platine, en or ou en carbone. L'enzyme est immobilisée sur ou au voisinage de la surface de l'électrode. Fréquemment, l'enzyme est associée à un médiateur redox qui permet le transfert d'électrons entre l'enzyme et l'électrode. Les médiateurs les plus utilisés dans le cas de la GOx sont le ferrocène, le ferrocynaure et les complexes d'osmium. L'électrode de travail et la contre-électrode sont mises en contact avec l'échantillon à tester et lorsqu'une tension est appliquée entre l'électrode de travail et la contre-électrode un courant électrique circule dans le circuit crée par les électrodes et l'échantillon à tester. Ce courant est induit par la réaction d'oxydation du glucose à l'électrode de travail qui est catalysée par l'enzyme et la valeur de ce courant est fonction de la concentration du glucose dans l'échantillon à tester.

L'analyse glycémique la plus courante consiste à effectuer un certain nombre de tests chaque jour ou chaque semaine en analysant un petit échantillon de sang [5,6], ce qui est incommode, peu confortable et entraîne donc une mauvaise acceptabilité par les patients. Par ailleurs, de tels tests ne tiennent pas compte des périodes de repos et entraînent des approximations sur les mesures. En outre, ce type de surveillance ne fournit pas d'informations en temps réel et ne peut donc pas prévenir les événements hypoglycémiants (<3,0 mM) et hyperglycémiants (> 11,1 mM) à l'avance.

Une surveillance autonome continue de la glycémie est cruciale pour comprendre les tendances, la direction et la fréquence des changements de celle-ci. Il existe des glucomètres interstitiels qui permettent aux diabétiques de suivre leur glycémie en temps réel avec des patchs placés sur la peau. Cependant, ces dispositifs doivent être remplacés toutes les deux semaines à cause de la dérive du biocapteur. En effet, la dérive dans le temps des biocapteurs enzymatique en général et ceux du glucose en particulier est le verrou majeur de développement de biocapteurs enzymatiques capables de garder leur sensibilité sur de long périodes sans avoir besoin d'être re-calibrés ou remplacés.

Un biocapteur enzymatique électrochimique se dégrade au fur et à mesure du temps. La stabilité décroissante des enzymes, la baisse de leurs activités catalytiques et la dégradation de l'électrode en sont la cause.

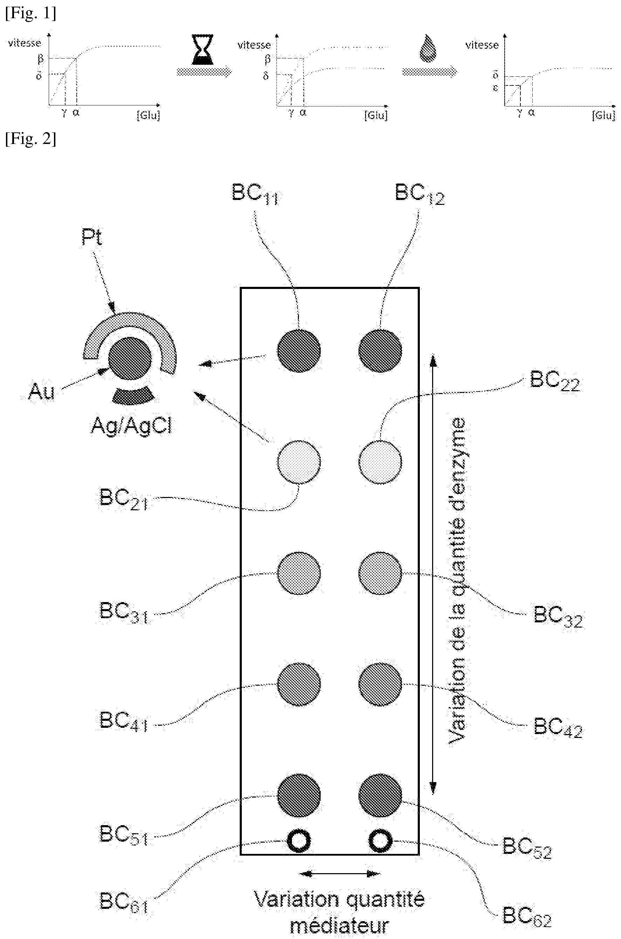

[Fig. 1] montre ce phénomène et son impact direct sur la quantification du glucose

Sur la [Fig. 1] sont représentés trois graphiques illustrant la vitesse de la réaction se produisant à l'électrode de travail d'un biocapteur électrochimique en fonction de la concentration du glucose. Sur le graphique de gauche, la concentration en glucose à une vitesse donnée est déterminée (les vitesses β et δ correspondent à des taux de glucose sanhuin (TGS) de α et γ). Au fil du temps, la dégradation enzymatique modifie les vitesses réactionnelles. Ceci est visible sur le graphique central de la [Fig. 1] : le TGS α n'est plus défini par la vitesse β mais par la vitesse δ. Les valeurs délivrées par le biocapteur ne sont donc plus fiables. Ainsi, une calibration est nécessaire, représentée par le graphique de droite. La calibration s'effectue de la manière suivante :
(1) L'utilisateur mesure son TGS par le biais d'une goutte de sang et transmet au biocapteur son TGS actuel égal à α.
(2) La vitesse δ mesurée par le capteur au même instant est enregistrée.
(3) Le programme contenu dans le capteur associant une vitesse à un TGS est réinitialisé avec les deux valeurs mesurées.

L'activité catalytique n'est pas restaurée. A partir d'un certain temps, le biocapteur aura perdu trop de précision et devra alors être remplacé.

Donc, pour tout biocapteur enzymatique la dérive de mesure est inévitable et une calibration externe est nécessaire. Pour cette raison le développement des biocapteurs à glucose implantables capables de fonctionner sur des longues périodes est aujourd'hui hors de portée. En outre d'une manière générale, pour tout biocapteur enzymatique cette dérive de la mesure est considérée comme étant la raison majeur pour leur non émergence dans le marché.

Les biocapteurs de glucose actuels destinés à mesurer le taux de glycémie sanguine sont limités par la dérive du biocapteur dans le temps ce qui constitue un verrou majeur pour le développement de biocapteurs à glucose implantable, ou non, capables de fonctionner sur de longues périodes de temps et de mesurer en continu le taux de glycémie chez les diabétiques.

Il existe donc un besoin pour un biocapteur enzymatique de glucose capable de « s'auto-calibrer » en s'affranchissant de la dérive de mesure des biocapteurs existants et de surveiller en continu la glycémie avec une stabilité à long terme sans besoin de remplacement ou de calibration externe. Ceci est réalisé en comparant les valeurs de mesure d'un ensemble de biocapteurs de caractéristiques connues et non sur la base des valeurs telles qu'elles sont mesurées.

Le document US 8,679,310 divulgue un biocapteur comprenant une première électrode de travail qui présente un premier biocatalyseur, une seconde électrode de travail qui présente un second biocatalyseur et une contre- électrode pour appliquer une tension à la première électrode de travail et à la seconde électrode de travail. La vitesse de réaction entre le premier biocatalyseur et le second biocatalyseur par rapport à une substance d'intérêt est différente. Le premier et le second biocatalyseur ont au moins une vitesse maximale différente ou une constante de Michaelis différente.

La présente invention porte sur un dispositif pour la mise en œuvre d'un procédé pour déterminer, de façon stable au cours du temps, une région dans laquelle se situe la concentration réelle, dans un milieu, d'un substrat (S₁) constitué par toute molécule susceptible de subir une oxydo-réduction catalysée par un catalyseur, caractérisé par le fait qu'il comprend au moins un groupe d'au moins deux biocapteurs, chaque biocapteur présentant une courbe d'étalonnage du signal induit par la réaction d'oxydo-réduction :
- les biocapteurs d'un groupe présentant des parties initiales identiques de leurs courbes d'étalonnage jusqu'à une valeur de concentration en substrat (S₁) dite concentration de séparation (CS) à partir de laquelle la mesure du signal diffère d'un biocapteur à l'autre du groupe ; et
- lorsque plus d'un groupe est présent, les biocapteurs des différents groupes présentant des courbes d'étalonnage différentes, sans présenter de parties initiales identiques entre les groupes, chaque biocapteur étant apte à mesurer un signal induit par une réaction d'oxydo-réduction catalytique du substrat (S1) et
chaque biocapteur comprenant :
- un catalyseur ;
- dans le cas d'un catalyseur enzymatique, le cas échéant un médiateur de ce dernier ; et
- le cas échéant un transporteur du substrat (S₁).

Comme les concentrations de séparation sont déterminées par comparaison successives entre deux courbes d'étalonnage, dans le cas d'un groupe comportant n biocapteurs et n courbes d'étalonnage correspondantes, il y a n-1 concentrations de séparation pour ce groupe.

Chaque biocapteur peut être apte à mesurer un signal électrochimique et comprend alors une électrode de travail, une électrode de référence et une contre-électrode entre lesquelles passe un courant induit par la réaction d'oxydation ou de réduction du substrat (S₁), le signal électrochimique étant soit l'intensité de ce courant, soit la différence de potentiel entre les électrodes lors de la réaction d'oxydo-réduction du substrat (S₁).

Pour chaque biocapteur, l'électrode de travail peut être une électrode en carbone, en or ou en platine, l'électrode de référence peut être une électrode en platine, en or ou en diamant et l'électrode de référence peut être une électrode au chlorure d'argent.

Le catalyseur peut être un catalyseur enzymatique ou un catalyseur chimique, le catalyseur chimique pouvant être un catalyseur abiotique choisi notamment parmi le platine, les nanostructures de platine, les nanostructures d'alliages de platine, les nanostructures d'or et les nanostructures d'alliages d'or, ou pouvant être un catalyseur moléculaire choisi notamment parmi un complexe de porphyrine-or et un complexe de porphyrine-rhodium.

Lorsque le catalyseur est un catalyseur enzymatique, un médiateur peut être associé audit catalyseur, ledit médiateur étant notamment choisi parmi le ferrocène, le ferrocyanure, les complexes d'osmium, les dérivés de quinone tels que la naphtoquinone, et les dérivés de phénothiazine.

Les biocapteurs de chaque groupe peuvent différer par au moins un paramètre choisi parmi :
p1 : la quantité de catalyseur ;
p2 : le Km d'oxydo-réduction du catalyseur, dans le cas où ce dernier est un catalyseur enzymatique ou la limite de saturation du catalyseur dans le cas où ce dernier est un catalyseur chimique ;
p3 : la quantité d'un médiateur du catalyseur, s'il est présent, dans le cas où celui-ci est un catalyseur enzymatique ; ou
p4 : le Km de transport d'un transporteur du substrat (S₁) s'il est présent.

Le Km d'oxydo-réduction du catalyseur enzymatique désigne la constante de Michaelis dudit catalyseur. Il représente la concentration en substrat pour laquelle la vitesse de la réaction est à la moitié de la vitesse maximale.

La constante de Michaelis d'un catalyseur enzymatique est spécifique de ce catalyseur et dépend de l'organisme dont ce catalyseur est issu et de son procédé d'extraction.

Par exemple, pour des enzymes glucose oxydase du commerce, la constante de Michaelis est de 1,34 mM pour une glucose oxydase provenant de *P. ostreatus,* de 5,7 mM pour une glucose oxydase provenant de *P. amagasakiense,* de 6,2 mM pour une glucose oxydase provenant de *P. pinophilum,* de 10,2 mM pour une glucose oxydase provenant de *T. flavus,* de 30 pour une glucose oxydase provenant de *A. niger* et de plus de de 38 mM pour une glucose oxydase provenant de *P. chrysosporium.*

La limite de saturation du catalyseur chimique est la concentration en substrat (S₁) à partir de laquelle la mesure du signal induit par la réaction d'oxydo-réduction du substrat (S₁) atteint une valeur limite maximale. Le Km est la concentration de substrat nécessaire pour que le signal induit par la réaction d'oxydo-réduction du substrat atteigne la moitié de la limite de saturation. On peut faire varier la limite de saturation car cette dernière est fonction de la nature du catalyseur chimique employé.

Le Km de transport d'un transporteur du substrat désigne la constante de Michaelis dudit transporteur. Il représente la concentration en substrat pour laquelle la vitesse de transport du substrat est à la moitié de la vitesse maximale.

A titre d'exemple, le Km de transport du transporteur de glucose Glu1 est de 3mM, celui de Glu2 est de 17 mM, celui de Glu3 est de 1,8mM, celui de Glu4 est de 5mM, celui de Glu8 est de 2,4 mM et celui de Glu9 est de 0,5 mM.

Les biocapteurs (BC) peuvent être notés :
BC₁₁..................BC₁₁..................BC₁ₙ...
BC₂₁..................BC₂ᵢ..................BC₂ₙ...
BCⱼ₁..................BCⱼᵢ..................BCⱼₙ...
BCₘ₁..................BCₘᵢ..................BCₘₙ...

m et n représentant chacun un nombre entier, m étant le nombre de groupes et n étant le nombre de biocapteurs dans chaque groupe, les biocapteurs BC₁₁ jusqu'à BC₁ₙ appartenant au groupe 1, les biocapteurs BCₘ₁ jusqu'à BCₘₙ appartenant au groupe m,
entre chaque biocapteur dans un groupe, on fait varier un paramètre choisi parmi p1 à p4 ; et entre les biocapteurs de deux groupes différents, on fait varier un autre de ces paramètres p1 à p4.

A titre d'exemple, on fait varier la quantité de médiateur du catalyseur entre chacun des biocapteurs d'un premier groupe afin que chaque biocapteur de ce groupe présente une courbe d'étalonnage différente.

Puis afin d'obtenir un deuxième groupe de biocapteurs différent, on peut conserver la même variation de la quantité de médiateur du catalyseur entre chacun des biocapteurs de ce second groupe, et pour créer une différence entre les premier et second groupe, on fait varier un second paramètre entre ces deux groupes, par exemple le Km d'oxydo-réduction du catalyseur. Le Km pourra par exemple être choisi à une première valeur pour chacun des biocapteurs dans le premier groupe et être choisi à une seconde valeur, différente de la première, pour chacun des biocapteurs dans le second groupe.

En particulier :
- le substrat (S₁) peut être le glucose ou le lactate ;
- le catalyseur peut être un catalyseur enzymatique choisi parmi une glucose oxydase, une glucose déshydrogénase et une cellobiose déshydrogénase, ou une lactate oxydase ou une lactate déshydrogénase ;
- le médiateur du catalyseur enzymatique, s'il est présent, peut être choisi parmi le ferrocène, le ferrocyanure, les complexes d'osmium, les dérivés de quinone tels que la naphtoquinone, et les dérivés de phénothiazine ;
- le transporteur du substrat (S₁), s'il est présent, peut être, le cas échéant, un transporteur de glucose qui est choisi notamment parmi GLUT1, GLUT2, GLUT3, GLUT4, GLUT6, GLUT8, GLUT10 et GLUT12, ou peut être un transporteur de lactate qui est choisi notamment parmi MCT1, MCT2, MCT3, MCT4.

Dans un mode de réalisation particulier, les biocapteurs peuvent être disposés sur un support, l'électrode de travail, l'électrode de référence et la contre-électrode étant imprimées par sérigraphie sur ledit support.

Le support sur lequel sont disposés les biocapteurs peut être choisi parmi les plaques de verre, de matière plastique, telle que le poly(téréphtalate d'éthylène), les plaques de céramique, telle que l'alumine ou un composite entre l'alumine et une autre céramique, les plaques de Nylon, les plaques de silicum, les films à base de polystyrène, ou les feuilles de polyester.

Le catalyseur peut être déposé sur l'électrode de travail de chaque biocapteur par encapsulation, greffage, absorption ou piégeage.

Lorsque le catalyseur est un catalyseur enzymatique, ce dernier peut être présent à la surface de l'électrode de travail de chaque biocapteur en étant appliqué sur ladite surface de l'électrode de travail à l'intérieur d'une couche protectrice, la couche protectrice étant notamment en chitosane, Nafion, polypyrrole, ou acide polyacrylique, on en un polymère conducteur tel que la polyaniline, l'acide polylactique, la polydopamine ou le polyéthylèneglycol. Le médiateur du catalyseur enzymatique peut être renfermé avec ledit catalyseur enzymatique à l'intérieur de la couche protectrice.

Un transporteur du substrat (S₁) peut être présent en étant appliqué en couche sur l'électrode de travail ou le cas échéant sur la couche protectrice comprenant le catalyseur enzymatique, son médiateur étant éventuellement présent, par dépôt d'une couche de protéoliposomes enfermant le transporteur du substrat (S₁).

Le support sur lequel sont disposés les biocapteurs et les biocapteurs peuvents être revêtus d'une couche de chitosane, de poly(méthacrylate de 2-hydroxyéthyle), de poly(4-vinylpyridine-co-styrène) ou d'alumine.

Le dispositif selon l'invention peut être disposé sur la peau d'un utilisateur ou peut être implanté dans l'utilisateur afin de déterminer une région dans laquelle se situe la concentration réelle du substrat (S₁), dans un milieu, ledit substrat étant le glucose et ledit milieu étant le sang.

En particulier, pour un dispositif implantable pour la détermination de la concentration en glucose, on peut choisir les biocapteurs de telle sorte qu'ils permettent de déterminer la concentration réelle en glucose dans une plage de concentration entre 2 et 10 mM avec une précision de 0,5 à 1 mM. C'est-à-dire que l'on choisira des biocapteurs avec des concentrations de séparation de 2 à 10 mM avec un pas de 0,5 ou 1 mM.

Les exemples suivants illustrent la présente invention sans toutefois en limiter la portée.

### Exemple 1 : Biocapteur « auto-calibré » basé sur la variation de la quantités de médiateur [M] et celle de l'enzyme [E]

Un réseau de 6 couples de biocapteurs est fabriqué à la surface d'une plaque de verre. On imprime des électrodes par sérigraphie sur la plaque de verre. Cette méthode de dépôt consiste à imprimer sur un support solide une électrode en carbone et cela à partir d'une encre de carbone en utilisant un appareil d'impression. Chaque biocapteur est composé d'une électrode de travail en carbone, d'une contre-électrode de platine et d'une électrode de référence de Ag/AgCl.

[Fig. 2] représente la disposition de l'électrode de travail, de la contre-électrode et de l'électrode de référence pour chaque biocapteur. L'électrode de travail de chaque biocapteur est fabriquée par sérigraphie à partir d'une encre de carbone contenant 40% en masse de poudre de carbone dispersée dans une solution organique composée de terpinéol et d'éthylcellulose. Les électrodes de référence et les contres électrodes de chaque biocapteur sont fabriquées par sérigraphie respectivement à partir d'une encre de platine contenant 40% en masse de poudre de platine dispersée dans une solution organique composée de terpinéol et d'éthylcellulose et d'une encre d'argent contenant 40% en masse de poudre d'argent dispersé » dans une solution organique composée de terpinéol et d'éthylcellulose.

Sur chaque électrode de travail, on dépose 10 µL d'une solution de Nafion-bromure de tétrabutylammonium (TBAB) (*tetrabutylammonium bromide Nafion)* à 5% en volume contenant l'enzyme glucose oxydase (GOx) et son médiateur naphtoquinone dans les quantités indiquées dans le Tableau 1 ci-après. On laisse tout sécher à l'air à la température ambiante pendant six heures.

On crée ainsi un ensemble de douze biocapteurs appartenant à six groupes de deux biocapteurs. Les biocapteurs au sein du premier groupe sont notés BC₁₁ et BC₁₂, les biocapteurs au sein du deuxième groupe BC₂₁ et BC₂₂, et BC₃₁ et BC₃₂ pour le troisième, BC₄₁ et BC₄₂ pour le quatrième, BC₅₁ et BC₅₂ pour le cinquième et BC₆₁ et BC₆₂ pour le sixième.

Au sein de chaque groupe de deux biocapteurs, les biocapteurs ont la même quantité d'enzyme GOx et présentent une quantité de médiateur différente. D'un groupe à l'autre, les quantités d'enzyme GOx et de médiateur changent.

On procède ensuite à un étalonnage des biocapteurs. Cet étalonnage initial consiste à mesurer l'intensité du courant induit par une réaction d'oxydo-réduction pour des solutions-étalons de glucose dont la concentration en glucose est connue. Le glucose est oxydé à l'électrode de travail alors que le dioxygène est réduit à la contre-électrode.

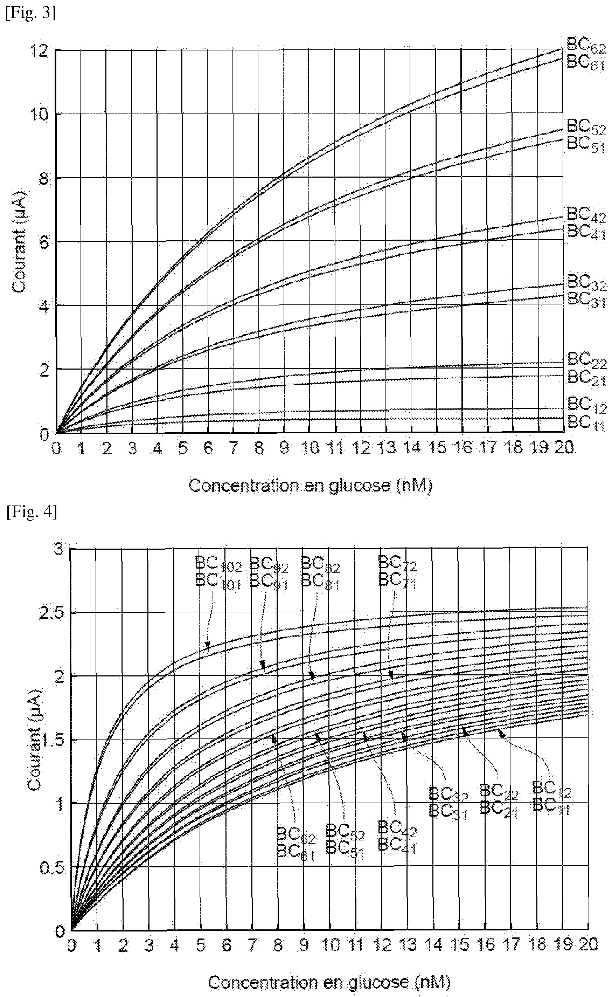

[Fig. 3] présente les courbes d'étalonnage que l'on a déterminé pour chacun des biocapteurs des six groupes. Ces courbes permettent d'obtenir les concentrations de séparation à partir desquelles, l'intensité du courant de la réaction d'oxydo-réduction du glucose devient différente entre les deux biocapteurs d'un même groupe.

Le tableau 1 présente également les valeurs de concentration de séparation (CS) relevées.

**[Tableaux1]**

| Biocapteurs | Concentration de séparation | Concentration en Glucose oxydase [E] | Concentration en naphtoquinone [M] |
|---|---|---|---|
| BC₁₁ | 1 mM | 0,02 mM | 7 mM |
| BC₁₂ | | | 10 mM |
| BC₂₁ | 2 mM | 0,01 mM | 11 mM |
| BC₂₂ | | | 13 mM |
| BC₃₁ | 3 mM | 0,007 mM | 13 mM |
| BC₃₂ | | | 15 mM |
| BC₄₁ | 4 mM | 0,005 mM | 17 mM |
| BC₄₂ | | | 19 mM |
| BC₅₁ | 5 mM | 0,004 mM | 20 mM |
| BC₅₂ | | | 22 mM |
| BC₆₁ | 6 mM | 0,003 mM | 22 mM |
| BC₆₂ | | | 24 mM |

Afin de déterminer la région de concentration en glucose d'un échantillon à analyser, on met en contact les groupes de biocapteurs avec ledit échantillon et on mesure la valeur du courant mesuré sur chaque biocapteur et on procède à la détermination de la valeur de la concentration en glucose selon la méthode suivante.

Si les valeurs mesurées aux deux biocapteurs BC₁₁ et BC₁₂ sont identiques ainsi que celles des autres biocapteurs, cela veut dire que la concentration en glucose dans l'échantillon est inférieure à la concentration de séparation de ce groupe de biocapteurs BC₁₁ et BC₁₂ qui est de 1 mM et est la plus faible concentration de séparation parmi les groupes.

Si les valeurs mesurées aux deux biocapteurs BC₁₁ et BC₁₂ sont différentes, cela veut dire que la concentration en glucose dans l'échantillon est supérieure à la concentration de séparation de ce groupe de biocapteurs BC₁₁ et BC₁₂ qui est de 1 mM.

On compare ensuite les valeurs mesurées sur le groupe de biocapteurs suivant, à savoir les biocapteurs BC₂₁ et BC₂₂. Si les valeurs mesurées aux deux biocapteurs BC₂₁ et BC₂₂ sont identiques, cela veut dire que la concentration en glucose dans l'échantillon est inférieure à la concentration séparation du groupe de biocapteurs BC₂₁ et BC₂₂ qui est de 2 mM. En faisant ainsi, on sait alors que la concentration en glucose dans l'échantillon est comprise entre 1 mM et 2 mM.

On procède de la même manière pour les biocapteurs suivants, à savoir si les valeurs mesurées aux biocapteurs BC₂₁ et BC₂₂ sont différentes et que les valeurs mesurées aux biocapteurs BC₃₁ et BC₃₂ sont identiques, alors la concentration en glucose se situe entre 2 mM et 3 mM, et ainsi de suite pour les groupes de biocapteurs suivants.

Si toutes les valeurs de tous les biocapteurs sont différentes, cela veut dire que la concentration en glucose dans l'échantillon est supérieure à la concentration de séparation la plus élevée qui est celle du dernier groupe de biocapteurs BC₆₁ et BC₆₂, à savoir 6 mM.

Le procédé permet donc d'encadrer la concentration en glucose en comparant les valeurs de l'intensité de la réaction d'oxydo-réduction du glucose mesurée au sein de chaque biocapteur pour chaque groupe.

Ce procédé permet de s'affranchir de la dérive des biocapteurs car ce ne sont plus les valeurs directement mesurées qui permettent d'établir la concentration en glucose mais la comparaison entre elles des valeurs mesurées à chaque biocapteur.

### Exemple 2 : Biocapteur « auto-calibré » basé sur la variation du Km de l'enzyme et la quantité de médiateur

Un ensemble de 10 groupes de deux biocapteurs est déposé à la surface d'une plaque de verre. Les électrodes de travail, contre-électrodes et électrodes de référence sont imprimées comme dans l'Exemple 1.

Sur chacune des électrodes de travail, on dépose 10 µL d'une solution de nafion-TBAB contenant 3mg/mL d'enzyme glucose oxydase et son médiateur naphtoquinone dans les quantités indiquées dans le tableau 2. Le Km des enzymes glucose oxydase varie selon les biocapteurs et est indiqué dans le tableau 2.

On crée ainsi un réseau de vingt biocapteurs appartenant à dix couples de biocapteurs. Ce réseau peut être noté comme suit :
BC₁₁............BC₁₂
BC₂₁............BC₂₂
BC₃₁............BC₃₂
BC₄₁............BC₄₂
BC₅₁............BC₅₂
BC₆₁............BC₆₂
BC₇₁............BC₇₂
BC₈₁............BC₈₂
BC₉₁............BC₉₂
BC₁₀₁............BC₁₀₂

Au sein de chaque couple, le Km de la glucose oxydase est le même et la quantité de médiateur est différente. D'un couple à l'autre, seul le Km de la glucose oxydase est différent.

[Fig. 4] présente les courbes d'étalonnage que l'on a déterminé pour chacun des biocapteurs des dix groupes. Ces courbes permettent d'obtenir les concentrations de séparation à partir desquelles, l'intensité du courant de la réaction d'oxydo-réduction du glucose devient différente entre les deux biocapteurs d'un même groupe.

**[Tableaux2]**

| Biocapteurs | Concentration de séparation | Km de la Glucose oxydase | Quantité de naphtoquinone (µg) |
|---|---|---|---|
| BC₁₁ | 4,5 mM | 5 | 18 |
| BC₁₂ | | | 20 |
| BC₂₁ | 4,3 mM | 10 | 18 |
| BC₂₂ | | | 20 |
| BC₃₁ | 3,8 mM | 15 | 18 |
| BC₃₂ | | | 20 |
| BC₄₁ | 3,5 mM | 20 | 18 |
| BC₄₂ | | | 20 |
| BC₅₁ | 3,1 mM | 25 | 18 |
| BC₅₂ | | | 20 |
| BC₆₁ | 2,7 mM | 30 | 18 |
| BC₆₂ | | | 20 |
| BC₇₁ | 2,2 mM | 35 | 18 |
| BC₇₂ | | | 20 |
| BC₈₁ | 1,8 mM | 40 | 18 |
| BC₈₂ | | | 20 |
| BC₉₁ | 1,3 mM | 45 | 18 |
| BC₉₂ | | | 20 |
| BC₁₀₁ | 1,2 mM | 50 | 18 |
| BC₁₀₂ | | | 20 |

On procède de la même manière qu'à l'Exemple 1 afin de déterminer la plage de concentration en glucose.

Par exemple, si toutes les valeurs des signaux des couples de biocapteurs BC₁₁, BC₁₂ ; BC₂₁, BC₂₂ ; BC₃₁, BC₃₂ ; BC₄₁, BC₄₂ ; BC₅₁, BC₅₂ ; sont identiques entre chaque couple, de biocapteurs, cela veut dire que la concentration en glucose est inférieure à la concentration de séparation la plus faible de ces couples. Ici, il s'agit de la concentration du couple BC₅₁, BC₅₂ qui est de 3,1 mM.

Les valeurs des signaux des autres biocapteurs sont donc différentes, ce qui veut dire que la concentration en glucose est supérieure à la concentration de séparation la plus élevée de ces couples. Ici, il s'agit de la concentration du couple BC61, BC62 qui est de 2,7 mM.

La concentration en glucose se trouve donc dans la plage de concentrations comprises entre 2,7 mM et 3,1 mM.

### Exemple 3 : Biocapteur « auto-calibré » biomimétique

Un groupe de quatre biocapteurs sont déposés à la surface d'une plaque de verre. Les électrodes de travail, contre-électrodes et électrodes de référence sont imprimées comme dans l'Exemple 1.

Sur chaque électrode de travail 1 en carbone, on dépose par électropolymérisation une couche 2 de polyaniline (PANI) à partir d'une solution tampon de phosphate de potassium (100 mM, pH 7) contenant 2mM d'aniline et 2mg/mL d'enzyme glucose oxydase (GOx) 3.

L'électropolymérisation est réalisée par voltamétrie cyclique entre -0,5 V et 1 V (5 cycles, vitesse de balayage 10mV/s) dans une cellule à trois électrodes avec un fil de platine comme contre-électrode et un fil d'argent comme électrode de référence.

Sur chaque électrode de travail sur laquelle on a déposé une couche de polyaniline, on dépose 100 µL d'une solution tampon de phosphate de potassium (100 mM, pH 7) contenant des protéoliposomes contenant des protéines transporteurs de glucose 5 avec un Km de 0,3 mM sur la première électrode de carbone, 0,6 mM sur la deuxième électrode de carbone, 2 mM sur la troisième électrode de carbone et de 5 mM sur la quatrième électrode de carbone. La concentration du solution tampon de phosphate en transporteur de glucose est de 0,11 mg/mL. Le diamètre des protéoliposomes 100 nm à 200 nm). On attend pendant 20 minutes pour permettre la fusion des protéoliposomes 4 à la surface de chacune des électrodes de travail.

La fusion des protéoliposomes 4 permet la formation d'une double couche lipidique 6 plane à la surface de l'électrode de travail et dans laquelle se trouve le transporteur de glucose 5.

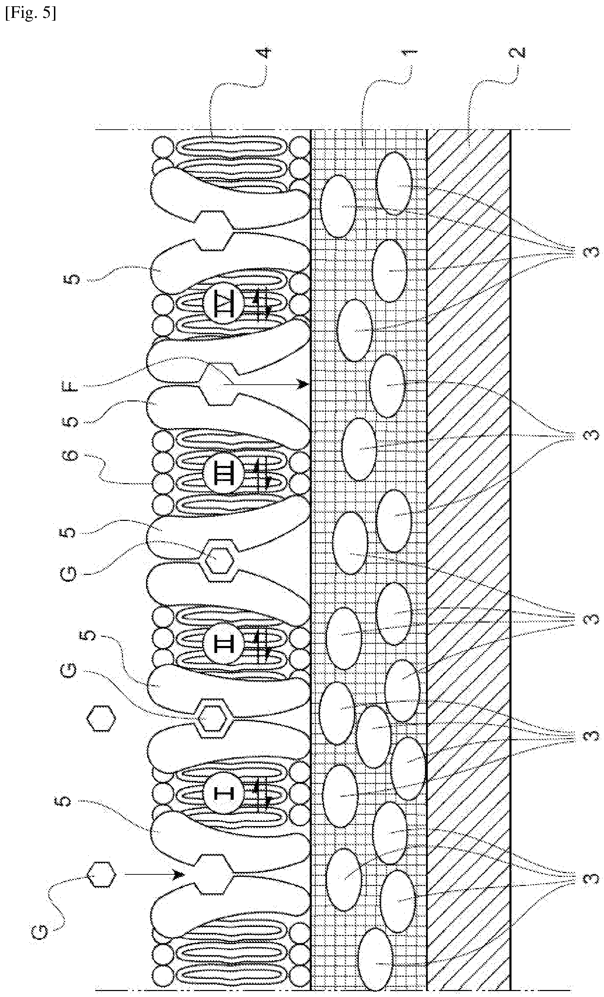

[Fig. 5] schématise une électrode de travail 1 d'un biocapteur ainsi que l'approche du glucose G vers la surface de l'électrode de travail 1. Dans une première étape I, Le glucose G s'approche de la protéine transporteur de glucose 5. Dans une étape II, le glucose G est capté par la protéine transporteur de glucose 5. Dans une étape III, le glucose G est libéré par la protéine transporteur de glucose 5 et se dirige vers la couche 2 de polyaniline dans laquelle se trouve l'enzyme GOx 3 dans le sens de la flèche F où le glucose G va être oxydé. Dans une étape IV, la protéine transporteur de glucose 5 est de nouveau prête à recevoir du glucose.

La protéine transporteur de glucose permet de moduler l'accès du glucose vers la glucose oxydase (Gox) déposée sur la surface de l'électrode de travail.

Par étalonnage, on peut déterminer la concentration de séparation de séparation entre les biocapteurs. Dans ce cas, les courbes d'étalonnage présentent sous la forme d'histogramme relevé pour chacun des biocapteurs. Dans ce cas, la concentration de séparation correspond à la concentration à partir de laquelle, la valeur d'intensité du courant atteint un palier pour un biocapteur, le transporteur de glucose ne pouvant plus transporter davantage de glucose vers l'électrode de travail.

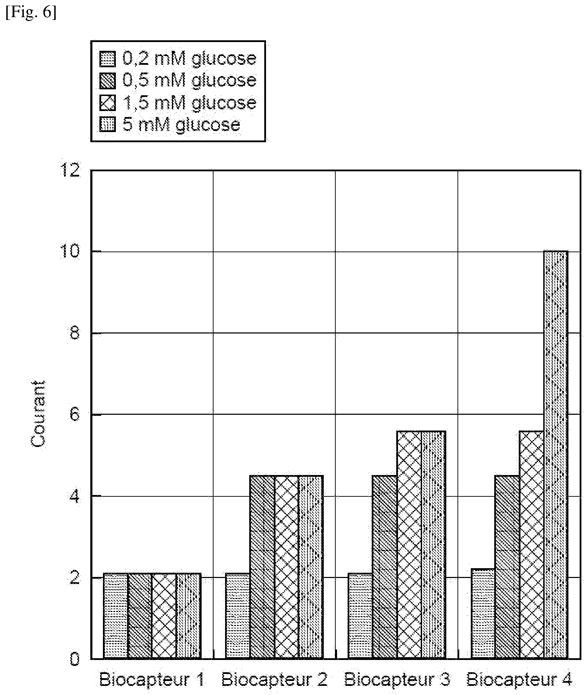

[Fig. 6] montre les courbes d'étalonnage des quatre biocapteurs de l'Exemple 3.

Le tableau 3 indique le Km du transporteur de glucose et les concentrations de séparation entre les biocapteurs.

**[Tableaux3]**

| Biocapteur | Km du transporteur de glucose | Concentration de séparation |
|---|---|---|
| 1 | 0,3 mM | 0,2 mM |
| 2 | 0,6 mM | 0,5 mM |
| 3 | 2 mM | 1,5 mM |
| 4 | 5 mM | - |

Afin de déterminer la région de concentration en glucose d'un échantillon à analyser, on met en contact les quatre biocapteurs avec ledit échantillon et on mesure la valeur du courant mesuré sur chaque biocapteur et on procède à la détermination de la valeur de la concentration en glucose selon la méthode suivante.

Si toutes les valeurs mesurées à chacun des biocapteurs sont égales, cela veut dire que la concentration en glucose est inférieure ou égale à la concentration de séparation la plus faible, à savoir 0,2 mM.

Si la valeur mesurée pour le premier biocapteur est différente de celles des autres biocapteurs et que les valeurs de chacun des trois autres biocapteurs sont identiques, la concentration en glucose est donc supérieure à la concentration de séparation du premier biocapteur et inférieure ou égale à la concentration de séparation du deuxième biocapteur. La concentration en glucose sera donc supérieure à 0,2 mM et inférieure ou égale à 0,5 mM.

Si toutes les valeurs mesurées par les biocapteurs sont différentes, cela veut dire la concentration en glucose est supérieure à la concentration de séparation du troisième biocapteur à savoir 1,5 mM.

### Exemple 4 : Biocapteur implantable

Un ensemble de 10 groupes de deux biocapteurs est déposé à la surface d'une plaque de silicium. Les électrodes de travail, contre-électrodes et électrodes de référence sont imprimées comme dans l'Exemple 1. L'enzyme glucose oxydase et son médiateur sont ensuite appliqués sur chaque électrode de travail dans les quantités indiquées à l'Exemple 1.

La plaque de silicium est ensuite recouverte d'une membrane biocompatible de chitosane par immersion dans une solution de chitosane à 2%, à température ambiante pendant 10s. Ensuite, on laisse sécher la plaque pendant 8 heures à 4°C.

On obtient ainsi un biocpateur qui peut être implanté sous la peau d'un utilisateur, afin de pouvoir mesurer la concentration en glucose dans le sang de l'utilisateur.

### Exemple 5 : Biocapteur « auto-calibré » pour la mesure de la concentration en lactate

Deux biocapteurs sont déposés à la surface d'une plaque de verre. Les électrodes de travail, contre-électrodes et électrodes de référence sont imprimées comme dans l'Exemple 1.

Sur chaque électrode de travail, on dépose 10 µL d'une solution de chitosane à 1% en volume contenant l'enzyme lactate oxydase (LOx) et son médiateur naphtoquinone dans les quantités indiquées dans le Tableau 4 ci-après. On laisse tout sécher à l'air à la température ambiante pendant six heures.

**[Tableaux4]**

| Biocapteurs | Concentration en lactate oxydase [E] | Concentration en naphtoquinone [M] |
|---|---|---|
| A1 | 0,04 mM | 1 mM |
| A2 | | 10 mM |

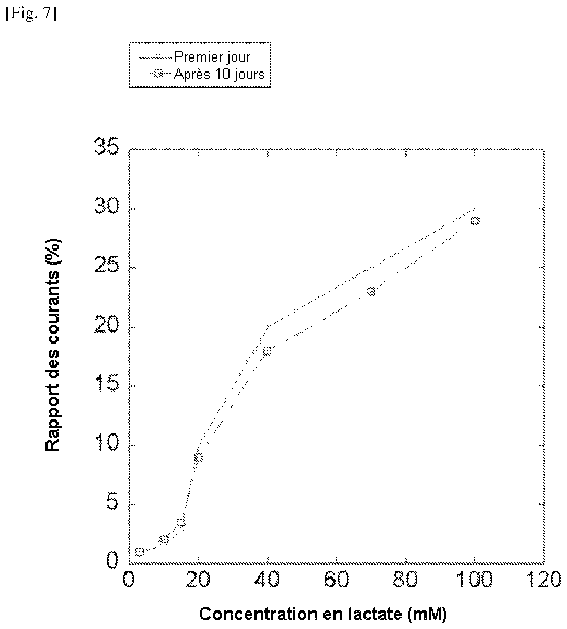

[Fig. 7] montre une courbe du rapport de l'intensité du courant mesurée pour les biocapteurs A1 et A2 en fonction de la concentration en lactate au cours du temps.

Comme on peut le voir sur la [Fig. 7], le rapport de l'intensité du courant mesurée pour les biocapteurs A1 et A2 est similaire au premier jour de la mesure et dix jours après la première mesure.

Ceci signifie que la dérive dans le temps des biocapteurs A1 et A2 est identique. Par conséquent, à l'aide d'un ensemble de biocapteurs, il et possible de déterminer la région de concentration en lactate d'un échantillon à analyser.

### Références

[1] Global report on diabetes (2016). World Health Organization
[2] International Diabetes federation (2016)
[3] Mechanisms of diabetic complications. Forbes JM1, Cooper ME. Physiol Rev. 2013 Jan ;93(1):137-88. doi: 10.1152/physrev.00045.2011
[4] Institut de veille sanitaire (France)
[5] Home Blood Glucose Biosensors: A Commercial Perspective. Jeffrey D. Newman & Anthony P.F. Turner. Biosensors and Bioelectronics, Volume 20, Issue 12, 20th Anniversary of Biosensors and Bioelectronics, 15 June 2005, Pages 2435-2453
[6] Electrochemical Glucose Biosensors. J. Wang. Chem. Rev., 2008, 108 (2), pp 814-825

## Revendications

1. - Dispositif pour la mise en œuvre d'un procédé pour déterminer, de façon stable au cours du temps, une région dans laquelle se situe la concentration réelle, dans un milieu, d'un substrat (S₁) constitué par toute molécule susceptible de subir une oxydo-réduction catalysée par un catalyseur, **caractérisé par le fait qu'**il comprend des groupes d'au moins deux biocapteurs, chaque biocapteur présentant une courbe d'étalonnage du signal induit par la réaction d'oxydo-réduction :
- les biocapteurs d'un groupe présentant des parties initiales identiques de leurs courbes d'étalonnage jusqu'à une valeur de concentration en substrat (S₁) dite concentration de séparation (CS) à partir de laquelle la mesure du signal diffère d'un biocapteur à l'autre du groupe ; et
- les biocapteurs des différents groupes présentant des courbes d'étalonnage différentes, sans présenter de parties initiales identiques entre les groupes, chaque biocapteur étant apte à mesurer un signal induit par une réaction d'oxydo-réduction catalytique du substrat (S1) et
chaque biocapteur comprenant :
- un catalyseur ;
- dans le cas d' un catalyseur enzymatique, le cas échéant un médiateur de ce dernier ; et
- le cas échéant un transporteur du substrat (S₁),
les biocapteurs de chaque groupe différant par au moins un paramètre choisi parmi :
p1 : la quantité de catalyseur ;
p2 : la constante de Michaelis du catalyseur, dans le cas où ce dernier est un catalyseur enzymatique ou la limite de saturation du catalyseur dans le cas où ce dernier est un catalyseur chimique ;
p3 : la quantité d'un médiateur du catalyseur, s'il est présent, dans le cas où celui-ci est un catalyseur enzymatique ; ou
p4 : la constante de Michaelis d'un transporteur du substrat (S1) s'il est présent ;
les biocapteurs (BC) étant notés :
BC₁₁..................BC₁ᵢ..................BC₁ₙ...
BC₂₁..................BC₂ᵢ..................BC₂ₙ...
BCⱼ₁..................BCⱼᵢ..................BCⱼₙ...
BCₘ₁..................BCₘᵢ..................BCₘₙ...
m et n représentant chacun un nombre entier, m étant le nombre de groupes et n étant le nombre de biocapteurs dans chaque groupe, les biocapteurs BC₁₁ jusqu'à BC₁ₙ appartenant au groupe 1, les biocapteurs BCₘ₁ jusqu' à BCₘₙ appartenant au groupe m,
entre chaque biocapteur dans un groupe, on fait varier un paramètre choisi parmi p1 à p4 ; et entre les biocapteurs de deux groupes différents, on fait varier un autre de ces paramètres p1 à p4.

2. - Dispositif selon la revendication 1, **caractérisé par le fait que** chaque biocapteur est apte à mesurer un signal électrochimique et comprend alors une électrode de travail, une électrode de référence et une contre-électrode entre lesquelles passe un courant induit par la réaction d'oxydation ou de réduction du substrat (S₁), le signal électrochimique étant soit l'intensité de ce courant, soit la différence de potentiel entre les électrodes lors de la réaction d'oxydo-réduction du substrat (S₁).

3. - Dispositif selon la revendication 2, **caractérisé par le fait que**, pour chaque biocapteur, l'électrode de travail est une électrode en carbone, en or ou en platine, l'électrode de référence est une électrode en platine, en or ou en diamant et l'électrode de référence est une électrode au chlorure d'argent.

4. - Dispositif selon l'une des revendications 2 à 3, **caractérisé par le fait que** le catalyseur est un catalyseur enzymatique ou un catalyseur chimique, le catalyseur chimique pouvant être un catalyseur abiotique choisi notamment parmi le platine, les nanostructures de platine, les nanostructures d'alliages de platine, les nanostructures d'or et les nanostructures d'alliages d'or, ou pouvant être un catalyseur moléculaire choisi notamment parmi un complexe de porphyrine-or et un complexe de porphyrine-rhodium.

5. - Dispositif selon l'une des revendications 1 à 4, dans lequel le catalyseur est un catalyseur enzymatique, **caractérisé par le fait qu'**un médiateur est associé audit catalyseur, ledit médiateur étant notamment choisi parmi le ferrocène, le ferrocyanure, les complexes d'osmium, les dérivés de quinone tels que la naphtoquinone, et les dérivés de phénothiazine.

6. - Dispositif selon l'une des revendications 1 à 5, **caractérisé par le fait que** :
- le substrat (S₁) est le glucose ;
- le catalyseur est un catalyseur enzymatique choisi parmi une glucose oxydase, une glucose déshydrogénase et une cellobiose déshydrogénase ;
- le médiateur du catalyseur enzymatique, s'il est présent, est choisi parmi le ferrocène, le ferrocyanure, les complexes d'osmium, les dérivés de quinone tels que la naphtoquinone, et les dérivés de phénothiazine ;
- le transporteur du substrat (S₁), s'il est présent, est un transporteur de glucose qui est choisi notamment parmi GLUT1, GLUT2, GLUT3, GLUT4, GLUT6, GLUT8, GLUT10 et GLUT12.

7. - Dispositif selon l'une des revendications 1 à 6, **caractérisé par le fait que** :
- le substrat (S₁) est le lactate ;
- le catalyseur est un catalyseur enzymatique choisi parmi une lactate oxydase ou une lactate déshydrogénase ;
- le médiateur du catalyseur enzymatique, s'il est présent, est choisi parmi le ferrocène, le ferrocyanure, les complexes d'osmium, les dérivés de quinone tels que la naphtoquinone, et les dérivés de phénothiazine ;
- le transporteur du substrat (S₁), s'il est présent, est un transporteur de lactate qui est choisi notamment parmi MCT1, MCT2, MCT3, MCT4.

8. - Dispositif selon l'une des revendications 1 à 7, **caractérisé par le fait que** les biocapteurs sont disposés sur un support, l'électrode de travail, l'électrode de référence et la contre-électrode étant imprimées par sérigraphie sur ledit support.

9. - Dispositif selon la revendication 8, **caractérisé par le fait que** le support sur lequel sont disposés les biocapteurs est choisi parmi les plaques de verre, de matière plastique, telle que le poly(téréphtalate d'éthylène), les plaques de céramique, telle que l'alumine ou un composite entre l'alumine et une autre céramique, les plaques de Nylon, les plaques de silicium, les films à base de polystyrène, ou les feuilles de polyester.

10. - Dispositif selon l'une des revendications 8 et 9, **caractérisé par le fait que** le catalyseur est déposé sur l'électrode de travail de chaque biocapteur par encapsulation, greffage, absorption ou piégeage.

11. - Dispositif selon la revendication 10, **caractérisé par le fait que** le catalyseur est un catalyseur enzymatique et est présent à la surface de l'électrode de travail de chaque biocapteur en étant appliqué sur ladite surface de l'électrode de travail à l'intérieur d'une couche protectrice, la couche protectrice étant notamment en chitosane, Nafion, polypyrrole, ou acide polyacrylique, on en un polymère conducteur tel que la polyaniline, l'acide polylactique, la polydopamine ou le polyéthylèneglycol.

12. - Dispositif selon la revendication 11, **caractérisé par le fait que** le médiateur du catalyseur enzymatique est renfermé avec ledit catalyseur enzymatique à l'intérieur de la couche protectrice.

13. - Dispositif selon l'une des revendications 8 à 12, **caractérisé par le fait qu'**un transporteur du substrat (S₁) est présent en étant appliqué en couche sur l'électrode de travail ou le cas échéant sur la couche protectrice comprenant le catalyseur enzymatique, son médiateur étant éventuellement présent, par dépôt d'une couche de protéoliposomes enfermant le transporteur du substrat (S₁).

14. - Dispositif selon l'une des revendications 8 à 13, **caractérisé par le fait que** le support et les biocapteurs portés par ce dernier sont revêtus d'une couche de chitosane, de poly(méthacrylate de 2-hydroxyéthyle), de poly(4-vinylpyridine-co-styrène) ou d'alumine.

15. - Dispositif selon l'une des revendications 8 à 14, **caractérisé par le fait qu'**il est disposé sur la peau d'un utilisateur ou qu'il est implanté dans l'utilisateur afin de déterminer une région dans laquelle se situe la concentration réelle du substrat (S₁), dans un milieu, ledit substrat étant le glucose et ledit milieu étant le sang.

## Patentansprüche

1. - Vorrichtung zum Durchführen eines Verfahrens zum Bestimmen, auf zeitlich stabile Weise im Verlauf der Zeit, eines Bereichs, in dem sich die tatsächliche Konzentration in einem Medium eines Substrats (S₁) befindet, das durch jedes Molekül gebildet ist, das einer durch einen Katalysator katalysierten Oxidoreduktion unterzogen werden kann, **dadurch gekennzeichnet, dass** sie Gruppen von mindestens zwei Biosensoren umfasst, wobei jeder Biosensor eine Kalibrierkurve des durch die Oxidoreduktionsreaktion induzierten Signals aufweist, wobei
- die Biosensoren einer Gruppe identische Anfangsteile ihrer Kalibrierkurven bis zu einem Substratkonzentrationswert (S₁) aufweisen, der als Trennkonzentration (CS) bezeichnet wird, ab dem sich die Signalmessung von einem Biosensor zum anderen der Gruppe unterscheidet; und
- die Biosensoren der verschiedenen Gruppen unterschiedliche Kalibrierkurven aufweisen, ohne zwischen den Gruppen identische Anfangsteile aufzuweisen, wobei jeder Biosensor geeignet ist, um ein durch eine katalytische Oxidoreduktionsreaktion des Substrats (S₁) induziertes Signal zu messen, und
jeder Biosensor umfassend:
- einen Katalysator,
- im Fall eines enzymatischen Katalysators optional einen Mediator davon; und
- optional einen Transporter des Substrats (S₁),
wobei sich die Biosensoren jeder Gruppe durch mindestens einen aus den folgenden ausgewählten Parameter unterscheiden:
p1: die Menge an Katalysator
p2: die Michaelis-Konstante des Katalysators, wenn dieser ein enzymatischer Katalysator ist, oder die Sättigungsgrenze des Katalysators, wenn dieser ein chemischer Katalysator ist
p3: die Menge eines Mediators des Katalysators, wenn er vorhanden ist, wenn dieser ein enzymatischer Katalysator ist; oder
p4: die Michaelis-Konstante eines Transporters des Substrats (S₁), wenn er vorhanden ist,
wobei die Biosensoren (BC) bezeichnet sind als
BC₁₁ ...............BC₁ᵢ ...............BC₁ₙ...
BC₂₁ ...............BC₂ᵢ ...............BC₂ₙ...
BCⱼ₁ ................BCⱼᵢ ...............BCⱼₙ...
BCₘ₁ ...............BCₘᵢ ..............BCₘₙ...
wobei m und n jeweils eine ganze Zahl darstellen, m die Anzahl der Gruppen und n die Anzahl der Biosensoren in jeder Gruppe ist, wobei die Biosensoren BC₁₁ bis BC₁ₙ zu der Gruppe 1 gehören, die Biosensoren BCₘ₁ bis BCₘₙ zu der Gruppe m gehören,
zwischen jedem Biosensor innerhalb einer Gruppe ein aus p1 bis p4 ausgewählter Parameter variiert wird; und
zwischen den Biosensoren von zwei verschiedenen Gruppen ein anderer dieser Parameter p1 bis p4 variiert wird.

2. - Vorrichtung nach Anspruch 1, **dadurch gekennzeichnet, dass** jeder Biosensor geeignet ist, um ein elektrochemisches Signal zu messen und dann eine Arbeitselektrode, eine Referenzelektrode und eine Gegenelektrode umfasst, zwischen denen ein durch die Oxidations- oder Reduktionsreaktion des Substrats (S₁) induzierter Strom fließt, wobei das elektrochemische Signal entweder die Stärke dieses Stroms oder die Potentialdifferenz zwischen den Elektroden während der Oxidoreduktionsreaktion des Substrats (S₁) ist.

3. - Vorrichtung nach Anspruch 2, **dadurch gekennzeichnet, dass** die Arbeitselektrode für jeden Biosensor eine Elektrode aus Kohlenstoff, Gold oder Platin ist, die Gegenelektrode eine Elektrode aus Platin, Gold oder Diamant ist und die Referenzelektrode eine Silberchloridelektrode ist.

4. - Vorrichtung nach einem der Ansprüche 2 bis 3, **dadurch gekennzeichnet, dass** der Katalysator ein enzymatischer Katalysator oder ein chemischer Katalysator ist, wobei der chemische Katalysator ein abiotischer Katalysator sein kann, der insbesondere ausgewählt ist aus Platin, Platin-Nanostrukturen, Platinlegierung-Nanostrukturen, Gold-Nanostrukturen und Goldlegierung-Nanostrukturen, oder ein molekularer Katalysator sein kann, der insbesondere ausgewählt ist aus einem Porphyrin-Gold-Komplex und einem Porphyrin-Rhodium-Komplex.

5. - Vorrichtung nach einem der Ansprüche 1 bis 4, wobei der Katalysator ein enzymatischer Katalysator ist, **dadurch gekennzeichnet, dass** mit dem Katalysator ein Mediator assoziiert ist, wobei der Mediator insbesondere ausgewählt ist aus Ferrocen, Ferrocyanid, Osmiumkomplexen, Chinonderivaten, wie beispielsweise Naphthochinon und Phenothiazinderivaten.

6. - Vorrichtung nach einem der Ansprüche 1 bis 5, **dadurch gekennzeichnet, dass**
- das Substrat (S₁) Glucose ist,
- der Katalysator ein enzymatischer Katalysator ist, ausgewählt aus einer Glucoseoxidase, einer Glucosedehydrogenase und einer Cellobiosedehydrogenase,
- der Mediator des enzymatischen Katalysators, wenn vorhanden, ausgewählt ist aus Ferrocen, Ferrocyanid, Osmiumkomplexen, Chinonderivaten, wie beispielsweise Naphthochinon und Phenothiazinderivaten,
- der Transporter des Substrats (S₁), wenn vorhanden, ein Glucosetransporter ist, der insbesondere ausgewählt ist aus GLUT1, GLUT2, GLUT3, GLUT4, GLUT6, GLUT8, GLUT10 und GLUT12.

7. - Vorrichtung nach einem der Ansprüche 1 bis 6, **dadurch gekennzeichnet, dass**
- das Substrat (S₁) das Lactat ist,
- der Katalysator ein enzymatischer Katalysator ist, ausgewählt aus einer Lactatoxidase oder einer Lactatdehydrogenase,
- der Mediator des enzymatischen Katalysators, wenn vorhanden, ausgewählt ist aus Ferrocen, Ferrocyanid, Osmiumkomplexen, Chinonderivaten, wie beispielsweise Naphthochinon und Phenothiazinderivaten,
- der Transporter des Substrats (S₁), wenn vorhanden, ein Lactattransporter ist, der insbesondere ausgewählt ist aus MCT1, MCT2, MCT3, MCT4.

8. - Vorrichtung nach einem der Ansprüche 1 bis 7, **dadurch gekennzeichnet, dass** die Biosensoren auf einem Träger angeordnet sind, die Arbeitselektrode, die Referenzelektrode und die Gegenelektrode durch Siebdruck auf den Träger gedruckt sind.

9. - Vorrichtung nach Anspruch 8, **dadurch gekennzeichnet, dass** der Träger, auf dem die Biosensoren angeordnet sind, ausgewählt ist aus Glasplatten, Platten aus Kunststoff, wie beispielsweise Polyethylenterephthalat, Keramikplatten, wie beispielsweise Aluminiumoxid oder einem Verbundstoff aus Aluminiumoxid und einer anderen Keramik, Nylonplatten, Siliciumplatten, Filmen auf Polystyrolbasis oder Polyesterfolien.

10. - Vorrichtung nach einem der Ansprüche 8 und 9, **dadurch gekennzeichnet, dass** der Katalysator auf der Arbeitselektrode von jedem Biosensor durch Einkapselung, Pfropfung, Absorption oder Einschluss aufgebracht wird.

11. - Vorrichtung nach Anspruch 10, **dadurch gekennzeichnet, dass** der Katalysator ein enzymatischer Katalysator ist und auf der Oberfläche der Arbeitselektrode von jedem Biosensor vorhanden ist, indem er auf die Oberfläche der Arbeitselektrode innerhalb einer Schutzschicht angewendet wird, wobei die Schutzschicht insbesondere aus Chitosan, Nafion, Polypyrrol oder Polyacrylsäure ist oder ein leitendes Polymer, wie beispielsweise Polyanilin, Polymilchsäure, Polydopamin oder Polyethylenglycol ist.

12. - Vorrichtung nach Anspruch 11, **dadurch gekennzeichnet, dass** der Mediator des enzymatischen Katalysators zusammen mit dem enzymatischen Katalysator innerhalb der Schutzschicht eingeschlossen ist.

13. - Vorrichtung nach einem der Ansprüche 8 bis 12, **dadurch gekennzeichnet, dass** ein Transporter des Substrats (S₁) vorhanden ist, indem er schichtförmig auf die Arbeitselektrode oder optional auf die den enzymatischen Katalysator umfassende Schutzschicht, sein möglichweise vorhandener durch Abscheiden einer Schicht von Proteoliposomen, die den Transporter des Substrats (S₁) einschließen, aufgebracht wird.

14. - Vorrichtung nach einem der Ansprüche 8 bis 13, **dadurch gekennzeichnet, dass** der Träger und die von diesem getragenen Biosensoren mit einer Schicht aus Chitosan, Poly(2-hydroxyethylmethacrylat), Poly(4-vinylpyridin-co-styrol) oder Aluminiumoxid beschichtet sind.

15. - Vorrichtung nach einem der Ansprüche 8 bis 14, **dadurch gekennzeichnet, dass** sie auf der Haut eines Benutzers aufgebracht wird ist oder in den Benutzer implantiert ist, um einen Bereich zu bestimmen, in dem sich die tatsächliche Konzentration des Substrats (S₁) in einem Medium befindet, wobei das Substrat Glucose und das genannte Medium Blut ist.

## Claims

1. - Device for implementing a method for determining, in a stable manner over the course of time, a region in which the actual concentration is located, in a medium, of a substrate (S₁) made up of any molecule likely to undergo catalyzed oxidation-reduction by a catalyst, **characterized by** the fact that it comprises groups of at least two biosensors, each biosensor having a calibration curve for the signal induced by the oxidation-reduction reaction:
- the biosensors in a group having identical initial portions of their calibration curves up to a concentration value of the substrate (S₁), referred to as the separation concentration (SC) from which the measurement of the signal differs from one biosensor in the group to another; and
- the biosensors in different groups having different calibration curves without having identical initial portions between the groups, each biosensor being able to measure a signal induced by a catalytic oxidation-reduction reaction of the substrate (S₁) and
each biosensor comprising:
- a catalyst;
- in the case of an enzymatic catalyst, if applicable, a mediator thereof; and
- if applicable a substrate (S₁) transporter,
the biosensors of each group differ in at least one parameter selected from:
p1: the amount of catalyst;
p2: the Michaelis constant of the catalyst, in case the latter is an enzymatic catalyst or the saturation limit of the catalyst in case the latter is a chemical catalyst;
p3: the amount of a mediator of the catalyst, if present, in case the catalyst is an enzymatic catalyst; or
p4: the Michaelis constant of a substrate (S₁) transporter if present
the biosensors (BC) are noted:
BC₁₁ ..................BC₁ᵢ ..................BC₁ₙ ...
BC₂₁ ..................BC₂ᵢ ..................BC₂ₙ ...
BCⱼ₁ ..................BCⱼᵢ ..................BCⱼₙ ...
BCₘ₁ ..................BCₘᵢ ..................BCₘₙ ...
m and n each being an integer, m being the number of groups and n being the number of biosensors in each group, the biosensors BC₁₁ to BC₁ₙ belonging to group 1, the biosensors BCₘ₁ to BCₘₙ belonging to group m,
between each biosensor in a group, a parameter selected from p1 to p4 is varied; and between the biosensors of two different groups, another of these parameters p1 to p4 is varied.

2. - Device according to claim 1, **characterized by** the fact that each biosensor is able to measure an electrochemical signal and then comprises a working electrode, a reference electrode and a counter-electrode between which passes a current induced by the oxidation or reduction reaction of the substrate (S₁), the electrochemical signal being either the intensity of this current or the potential difference between the electrodes during the oxidation-reduction reaction of the substrate (S₁).

3. - Device according to claim 2, **characterized by** the fact that for each biosensor the working electrode is a carbon, gold or platinum electrode, the reference electrode is a platinum, gold or diamond electrode and the reference electrode is a silver chloride electrode.

4. - Device according to one of claims 2 to 3, **characterized by** the fact that the catalyst is an enzymatic catalyst or a chemical catalyst, it being possible for the chemical catalyst to be an abiotic catalyst chosen in particular from platinum, platinum nanostructures, platinum alloy nanostructures, gold nanostructures and gold alloy nanostructures, or to be a molecular catalyst chosen in particular from a porphyrin-gold complex and a porphyrin-rhodium complex.

5. - Device according to one of claims 2 to 4, in which the catalyst is an enzymatic catalyst, **characterized by** the fact that a mediator is associated with the said catalyst, the said mediator being chosen in particular from ferrocene, ferrocyanide, osmium complexes, quinone derivatives such as naphthoquinone, and phenothiazine derivatives.

6. - Device according to one of claims 1 to 5, **characterized by** the fact that:
- the substrate (S₁) is glucose;
- the catalyst is an enzymatic catalyst selected from a glucose oxidase, a glucose dehydrogenase and a cellobiose dehydrogenase;
- the enzymatic catalyst mediator, if present, is selected from ferrocene, ferrocyanide, osmium complexes, quinone derivatives such as naphthoquinone, and phenothiazine derivatives;
- the substrate (S₁) transporter, if present, is a glucose transporter which is selected in particular from GLUT1, GLUT2, GLUT3, GLUT4, GLUT6, GLUT8, GLUT10 and GLUT12.

7. - Device according to one of claims 1 to 6, **characterized by** the fact that:
- the substrate (S₁) is lactate;
- the catalyst is an enzymatic catalyst selected from a lactate oxidase or a lactate dehydrogenase;
- the enzymatic catalyst mediator, if present, is selected from ferrocene, ferrocyanide, osmium complexes, quinone derivatives such as naphthoquinone, and phenothiazine derivatives;
- the substrate (S₁) transporter, if present, is a lactate transporter which is chosen in particular from MCT1, MCT2, MCT3, MCT4.

8. - Device according to one of claims 1 to 7, **characterized by** the fact that the biosensors are arranged on a support, the working electrode, the reference electrode and the counter-electrode being screen-printed on said support.

9. - Device according to claim 8, **characterized by** the fact that the support on which the biosensors are arranged is chosen from glass plates, plastic plates, such as polyethylene terephthalate plates, ceramic plates, such as alumina or a composite between alumina and another ceramic, nylon plates, silicon plates, polystyrene-based films, or polyester sheets.

10. - Device according to one of claims 8 and 9, **characterized by** the fact that the catalyst is deposited on the working electrode of each biosensor by encapsulation, grafting, absorption or trapping.

11. - Device according to claim 10, **characterized by** the fact that the catalyst is an enzymatic catalyst and is present on the surface of the working electrode of each biosensor by being applied on said surface of the working electrode inside a protective layer, the protective layer being in particular of chitosan, Nafion, polypyrrole, or polyacrylic acid, or of a conductive polymer such as polyaniline, polylactic acid, polydopamine or polyethylene glycol.

12. - Device according to claim 11, **characterized by** the fact that the enzymatic catalyst mediator is enclosed with said enzymatic catalyst within the protective layer.

13. - Device according to one of claims 8 to 12, **characterized by** the fact that a substrate (S₁) transporter is present by being applied as a layer to the working electrode or optionally on the protective layer comprising the enzymatic catalyst, its mediator optionally being present, by depositing a layer of proteoliposomes enclosing the substrate (S₁) transporter.

14. - Device according to one of claims 8 to 13, **characterized by** the fact that the support and the biosensors carried by the latter are coated with a layer of chitosan, poly(2-hydroxyethyl methacrylate), poly(4-vinylpyridine-co-styrene) or alumina.

15. - Device according to one of claims 8 to 14, **characterized by** the fact that it is arranged on the skin of a user or is implanted in the user in order to determine a region in which the actual concentration of the substrate (S₁) in a medium is located, said substrate being glucose and said medium being blood.
